(19) **Europäisches Patentamt**
**European Patent Office**
**Office européen des brevets**

(11) **EP 2 006 295 A2**

(12) **EUROPEAN PATENT APPLICATION**
published in accordance with Art. 153(4) EPC

(43) Date of publication:
**24.12.2008 Bulletin 2008/52**

(51) Int Cl.:
*C07K 14/435* $^{(2006.01)}$    *A23J 1/04* $^{(2006.01)}$
*A23J 3/04* $^{(2006.01)}$    *A23L 3/36* $^{(2006.01)}$
*A23B 4/08* $^{(2006.01)}$    *A23L 1/10* $^{(2006.01)}$
*A23L 1/16* $^{(2006.01)}$    *A23L 1/32* $^{(2006.01)}$
*A23L 1/325* $^{(2006.01)}$

(21) Application number: **07738454.3**

(22) Date of filing: **13.03.2007**

(86) International application number:
**PCT/JP2007/054982**

(87) International publication number:
**WO 2007/105731 (20.09.2007 Gazette 2007/38)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.03.2006 JP 2006067758**

(71) Applicant: **NIPPON SUISAN KAISHA, LTD.**
**Tokyo 100-8686 (JP)**

(72) Inventors:
• **CHIBA, Satoru**
**Hachioji-shi**
**Tokyo 192-0906 (JP)**

• **KUBOTA, Mitsutoshi**
**Hachioji-shi**
**Tokyo 192-0906 (JP)**
• **NISHIZAWA, Satoko**
**Hachioji-shi**
**Tokyo 192-0906 (JP)**
• **OKAMOTO, Naoko**
**Hachioji-shi**
**Tokyo 192-0906 (JP)**
• **SUZUKI, Kenichi**
**Hachioji-shi**
**Tokyo 192-0906 (JP)**

(74) Representative: **Vossius & Partner**
**Siebertstrasse 4**
**81675 München (DE)**

(54) **PROTEIN HAVING ICE NUCLEATING ACTIVITY**

(57)    A crustacean-derived protein, as measured by sodium dodecyl sulfate (SDS) - polyacrylamide gel electrophoresis, displays a non-reduced band of a molecular weight of about 200,000, displays reduced-form bands at about 86,000 and 90,000 molecular weights, and has N-terminal amino acids as indicated by SEQ ID No. 1 or SEQ ID No. 2.
This protein is contained in large amounts in the crustacean shells that are largely discarded except for use for meal, chitin, and chitosan raw material. Thus large volume preparation, as well as inexpensive production, is possible. Moreover, application by addition to foods is easy since this is a food-derived protein. Since the protein of the present invention has ice nucleation activity and recrystallization inhibition activity for ice crystals, wide use is possible with the object of quality maintenance and the like of frozen food and the like.

EP 2 006 295 A2

**Description**

[TECHNICAL FIELD]

The present invention relates to a protein that has ice nucleation activity. The present invention particularly relates to a protein that has ice nucleation activity and has recrystallization inhibition activity for ice crystals, a production method for the protein, and use of the protein.

[BACKGROUND ART]

Although water is said to become ice at 0°C, more specifically, the phenomenon of supercooling is known to occur when pure water is cooled to 0°C without the beginning of freezing, and where freezing does not occur even at temperatures below the freezing point. When the temperature becomes less than or equal to 0°C, some substances present in the water become nuclei for ice, and freezing of water proceeds by the process of crystal growth. If this freezing phenomenon can be controlled, applications are thought to be possible in fields beginning with foodstuff freezing preservation technology, microorganism and biological tissue storage technology, and the like as well as various fields such as artificial rainfall stimulation technology, cryogenic shipping technology, and the like.

Attention has been paid to proteins related to freezing of water, and such proteins are ice nucleation proteins (INP) and antifreeze proteins (AFP).

Ice nucleation activity bacteria are bacteria that have the ability to cause pure water, which does not freeze even at -20°C, to freeze at -2°C to -4°C, and thus ice nucleation activity bacteria clearly act as nuclei during the start of freezing of water. Such bacteria are known to cause frost damage in plants. Bacteria known to be such ice nucleating bacteria belong to the genus Pseudomonas and the genus Erwinia (Non-Patent Documents 1 and 2). An ice nucleation protein is a protein that is obtained from these ice nucleating bacteria and has ice nucleation activity. The majority of ice nucleation proteins are derived from microorganisms, and although their utilization has been proposed in the field of foods (Patent Documents 1 - 4), these ice nucleation proteins have actually not been put into much practical use.

[Patent Document 1] Unexamined Laid-Open Patent Application No. H6-181729

[Patent Document 2] Patent No. 3028246

[Patent Document 3] Unexamined Laid-Open Patent Application No. H6-113712

[Patent Document 4] Patent No. 3090932

[Non-Patent Document 1] Appl. Microbiol. 28, p. 456, (1974) [Non-Patent Document 2] Proc. 4th Int. Cont. Plant. Path. Bact., p. 725, (1978)

[DISCLOSURE OF THE INVENTION]

[PROBLEM TO BE SOLVED BY THE INVENTION]

The problem for the present invention is to provide a substance having ice nucleation activity that is capable of use in a wide range of fields, beginning with foods, from the standpoints of safety, activity, production rate, cost, and the like.

[MEANS FOR SOLVING THE PROBLEM]

Although experiments were started based on the thought of the inventors of the present invention that antifreeze protein might be contained in krill which inhabit the Antarctic ocean since such antifreeze proteins have been known from many types of organisms, a substance having antifreeze activity could not be obtained by methods such as those mentioned in commonly known references. By devising an extraction method, a protein was discovered that had antifreeze activity and a protein was also discovered that had ice nucleation activity.

Furthermore, as a result of paying attention also to non-krill crustaceans, the inventors of the present invention succeeded in obtaining similar substances by adoption of the same extraction method.

Furthermore, the present invention was completed by the discovery that the protein having ice nucleation activity had recrystallization inhibition activity for ice crystals.

The present invention provides methods (1) and (2) for inhibition of recrystallization of ice crystals:

(1) a method for inhibition of recrystallization of ice crystals utilizing a protein having ice nucleation activity; and

(2) the method for inhibition of recrystallization of ice crystals according to (1), wherein the protein having ice nucleation activity is a protein derived from a crustacean.

The present invention provides proteins (3) - (5) having ice nucleation activity:

(3) a crustacean-derived protein having ice nucleation activity;
(4) the protein having ice nucleation activity according to (3), wherein the crustacean-derived protein, as measured by molecular weight measurement using sodium dodecyl sulfate (SDS) - polyacrylamide gel electrophoresis, has a non-reduced-form molecular weight of about 200,000 and displays reduced-form bands at about 86,000 and 90,000 molecular weights, and the N-terminal amino acids are indicated by SEQ ID No. 1 or SEQ ID No. 2; and
(5) the protein having ice nucleation activity according to (3) or (4), wherein the crustacean is a shrimp, crayfish, crab, krill, or mantis shrimp.

The present invention provides crustacean extracts (6) - (8) having ice nucleation activity and including the protein having ice nucleation activity:

(6) a crustacean extract having ice nucleation activity and including a crustacean-derived protein having ice nucleation activity;
(7) the crustacean extract having ice nucleation activity according to (6), wherein the crustacean-derived protein having ice nucleation activity, as measured by molecular weight measurement using sodium dodecyl sulfate (SDS) - polyacrylamide gel electrophoresis, has a non-reduced-form molecular weight of about 200,000 and displays reduced-form bands at about 86,000 and 90,000 molecular weights, and the N-terminal amino acids are indicated by SEQ ID No. 1 or SEQ ID No. 2; and
(8) the crustacean extract having ice nucleation activity according to (6) or (7), wherein the crustacean is a shrimp, crayfish, crab, krill, or mantis shrimp.

The present invention provides production methods (9) - (12) for a crustacean extract having ice nucleation activity:

(9) a production method for a crustacean extract having crustacean-derived ice nucleation activity, wherein a liquid obtained by crushing meat and/or internal organs of the crustacean or an extract liquid of the shell, meat, and/or internal organs is utilized with or without further purification;
(10) a production method of a crustacean extract having crustacean-derived ice nucleation activity according to (9), wherein purification is used to concentrate a protein, as measured by molecular weight measurement using sodium dodecyl sulfate (SDS) - polyacrylamide gel electrophoresis, having a non-reduced-form molecular weight of about 200,000 and displaying reduced-form bands at about 86,000 and 90,000, and having the N-terminal amino acids as indicated by SEQ ID No. 1 or SEQ ID No. 2;
(11) the production method of a crustacean extract having crustacean-derived ice nucleation activity according to (9) or (10), wherein an extraction liquid containing added surfactant is used for extraction from the shell, meat, and/or internal organs of the crustacean; and
(12) the production method of a crustacean extract having crustacean-derived ice nucleation activity according to (9), (10), or (11), wherein the crustacean is a shrimp, crayfish, crab, krill, or mantis shrimp.

The present invention provides methods (13) - (17) for suppression of quality deterioration due to freezing of food by the suppressing of recrystallization of ice crystals of:

(13) a method for suppression of quality deterioration due to freezing of food by suppressing recrystallization of ice crystals, wherein a protein is added that has ice nucleation activity;
(14) the method of (13), wherein the protein of any one of (3) through (5) is added as the protein having ice nucleation activity, or the crustacean extract of any one of (6) through (8) is added;
(15) the method of (13) or (14), wherein the food is a frozen food;
(16) the method according to (13), (14), or (15), wherein the frozen food is a food utilizing egg, wheat flour, or fish meat as a raw material;
(17) the method according to (16), wherein the frozen food is surimi or minced fish meat.

The present invention provides food quality improvement agents, which includes the protein having ice nucleation activity or a crustacean extract having ice nucleation activity, and foods to which has been added this quality improvement agent, (18) - (21).

(18) a food quality improvement agent including: the protein having ice nucleation activity according to any one of (1) through (5), the crustacean extract having ice nucleation activity according to any one of (6) through (8), or the protein having ice nucleation activity produced by the method of any one of (9) through (12);

(19) a food having improved quality and to which has been added the quality improvement agent of (18);

(20) the food according to (19), wherein the food is a frozen food; and

(21) the food according to (19) or (20), wherein the food is a food utilizing egg, wheat flour, or fish meat as a raw material.

[ADVANTAGES OF THE INVENTION]

The protein that has ice nucleation activity of the present invention is a crustacean-derived protein that is suitable for addition to foods, and thus addition is possible to various types of foods. Although uses of a protein having ice nucleation activity have been proposed that utilize the property of easy freezing rather than just preventing supercooling, application to foods has been extremely limited. However, according to the present invention, recrystallization inhibition activity for ice crystals was discovered for a protein that has ice nucleation activity, and quality deterioration due to freezing could be suppressed by addition of this protein to foods undergoing freeze processing during the food production process.

[BRIEF DESCRIPTION OF THE DRAWINGS]

FIG. 1 shows the chromatography chart of the protein extracted from krill of Working Example 1.

FIG. 2 shows the Sephacryl S-200 HR gel filtration column chromatography chart of Working Example 1.

FIG. 3 is a photograph of electrophoresis using SDS-polyacrylamide gel to check molecular weight of the present protein sample 1 during Working Example 2.

FIG. 4 shows measurement results of ice nucleation activity of the present protein sample 1 of Working Example 3.

FIG. 5 shows temperature stability of ice nucleation activity of the present protein sample 1 of Working Example 3.

FIG. 6 shows recrystallization inhibition activity for ice crystals of the present protein sample 1 of Working Example 4.

FIG. 7 is a photograph of electrophoresis of the krill hot water extract of Working Example 7.

FIG. 8 shows salt-induced solubility of minced meat to which the present protein sample 1 had been added during Working Example 8.

FIG. 9 shows generation amount of DMA by minced meat to which the present protein sample 1 had been added during Working Example 8.

FIG. 10 shows the freeze temperature curve when the present protein sample 1 was added to chicken egg during Working Example 9.

FIG. 11 shows thermal history during freezing and thawing of the surimi containing the added present protein sample 1 during Working Example 10.

FIG. 12 shows the freeze curve when the present protein sample 1 is added to the heated gel of the surimi of Working Example 10.

[BEST MODE FOR CARRYING OUT THE INVENTION]

In the present invention, a protein having ice nucleation activity (referred to hereinafter as an ice nucleation protein) means a protein that has the effect of suppressing supercooling during freezing of water, and microorganism-derived proteins are known that are such proteins. The ice nucleation activity catalyzes the freezing of ice, and thus ice nucleation activity causes the supercooled state of water to end at a higher temperature below the freezing point. Although ice nucleation activity is used for the production of artificial snow and the like, application of ice nucleation activity is also being investigated for frozen foods. Anticipated applications that have been proposed (cryoconcentration of liquid foods, freeze drying, and the like) are based on the property which is the ability to suppress supercooling or are based on saving energy due to the ability to freeze at a higher temperature. However, use for such applications has not progressed due to concerns for safety and the like when utilizing a microorganism-derived protein.

The inventors of the present invention discovered that the ice nucleation protein not only suppresses supercooling but also had recrystallization inhibition activity for ice crystals. When frozen water is slightly melted by a rise of temperature, phenomena occur such as the frozen water freezing again around residual ice crystals such that the ice crystals gradually grow larger and the small ice crystals bond together to form large ice crystals. These phenomena are called recrystallization of ice crystals. The expression "recrystallization inhibition activity for ice crystals" is taken to mean activity that suppresses this recrystallization. The inventors of the present invention discovered that an ice nucleation protein had the properties of suppressing growth and bonding of ice crystals in this manner and maintaining ice crystals at a small unchanged size. By discovery of these properties, suppression of food quality deterioration due to temperature change during frozen storage is possible, not just during freezing of the frozen food and the like, and the range of applications can be expanded due to increase in the value of use of the ice nucleation protein.

In the present invention, the presence or absence of recrystallization inhibition activity for ice crystals was determined by observation of ice crystals using a microscope and observing the count of fine ice crystals. That is to say, 2 $\mu$L of a protein sample having recrystallization inhibition activity for ice crystals dissolved in a 30% sucrose solution is placed drop-wise on a cover glass, and another cover glass is placed thereon to sandwich the solution therebetween. The cover glass assembly is placed on the stage of an optical microscope (model BH2 microscope, produced by Olympus Corp.; and LK600 temperature controller, produced by Linkam Scientific Instruments Ltd.) that is capable of temperature control, and heating and cooling are performed repeatedly at a rate of 0.1°C/second, in order, to temperatures of 20°C, -30°C, 20°C, -30°C, 20°C, -30°C, and -10°C. Then temperature is held at -10°C for 30 minutes, and the count is determined of fine ice crystals having a surface area of 10.01 to 35 $\mu$m$^2$. Although ice crystals grow large and the number of fine ice crystals becomes small when there is no addition of a substance that has recrystallization inhibition activity for ice crystals, the number of fine ice crystals becomes high when there is recrystallization inhibition activity for ice crystals.

The inventors of the present invention discovered the presence of ice nucleation activity in an extract from a crustacean. Although use of ice nucleation proteins derived from microorganisms has been considerably delayed from the standpoint of safety, the crustacean itself is a food, and the ice nucleation protein of the present invention can be used for foods without concern. This ice nucleation activity is possessed by a liquid obtained by crushing crustacean meat, internal organs, and the like as well as by an extraction liquid obtained by extraction of meat, internal organs, and shells by a solvent such as water and the like. As shown in the working examples, this activity is stable at a temperature of about 0°C to 90°C, and thus wide use is possible in foods.

In the present invention, ice nucleation activity was evaluated as the extent of elevation of the temperature of freezing of water due to addition of the protein. Specifically, 100 $\mu$L of protein solution is added to 10 mL of tap water, and the mixture is incubated for 10 minutes in a cooling constant temperature tank set at 5°C. Thereafter, temperature is lowered at a rate of 0.3°C/minute, and the freezing temperature (supercooling temperature) is measured. The ice nucleation activity is determined to be higher as the freezing temperature increases.

The term "crustacean" in the present invention indicates the Crustacea class of the Pancrustacea subphylum of the Arthropoda phylum, and this term further indicates an animal classified as belonging to the Cepharocarid subclass, Remipedia subclass, Branchiopoda subclass, Maxillopoda subclass, or Malacostraca subclass. Examples that can be cited are: shrimps, crayfishes, and crabs included in the order Decapoda; northern krill and Antarctic krill of the order Euphausiacea; mantis shrimps of the order Stomatopoda; and the like, which are frequently used as food in the seafood industry.

In order to purify and concentrate the protein having ice nucleation activity of the present invention, general methods can be used for extraction, separation, and concentration of proteins. That is to say, all the tissue present in the crustacean is crushed, the obtained suspension liquid is subjected to centrifugal separation, and insoluble substances are removed. At this time, solubilization is accelerated and extraction ability is increased by use of a surfactant (cationic type, non-ionic type, amphoteric type, anionic type, high molecular weight surfactant and the like). A non-ionic surfactant is particularly suitable. Protein can be separated and concentrated from the obtained supernatant by the following indicated general methods: separation and concentration methods using ammonium sulfate fractionation as a salting-out method; methods using acetone, ethanol, propanol, or methanol for separation and concentration by an organic solvent; acidic precipitation methods using hydrochloric acid, sulfuric acid, or trichloroacetic acid; separation and concentration methods utilizing a water soluble polymer (polyethylene glycol or dextran); separation and concentration methods using ultrafiltration (membrane concentration); and adsorption and separation on an ion exchange chromatography, hydrophobic interaction chromatography, hydroxyapatite chromatography, reversed phase chromatography, or gel filtration chromatography. The supernatant can also be subjected to heat treatment to cause denaturation and precipitation of other proteins. The obtained isolate can be made into a powder by freeze drying, spray drying, and the like.

Specifically, the protein of the present invention is contained in the meat, internal organs, and shells. Thus, such raw material in the frozen, raw, or dried form is pulverized using a homogenizer, water is added to form a suspension, and extraction is performed for 30 minutes at about 50°C. When this extract is subjected to centrifugal treatment (3 minutes at 15,000 G), an ice nucleation protein solution is obtained as the supernatant. The extraction temperature is preferably 0°C to 80°C, and an extraction temperature of 0°C to 60°C is particularly preferred since there is a tendency for extraction efficiency to decline as the temperature is raised. This crude extraction liquid can be used without further processing since the crude extraction liquid itself has ice nucleation activity. In order to perform concentration and purification, a combination of the above mentioned protein purification and concentration methods can be used while utilizing as an indicator of purification and concentration a "protein having, as measured by molecular weight measurement using sodium dodecyl sulfate (SDS) - polyacrylamide gel electrophoresis, a non-reduced-form molecular weight of about 200,000 and displaying reduced-form bands at about 86,000 and 90,000, wherein the N-terminal amino acids are indicated by SEQ ID No. 1 or SEQ ID No. 2."

The ice nucleation protein of the present invention has recrystallization inhibition activity for ice crystals. Thus use is possible in a food that includes a freezing step during the production process or for control of quality deterioration due to freezing of a frozen food and the like. Suppression of quality deterioration is possible in foods having raw materials that have particularly low tolerance of freezing, such as egg, fish meat, wheat flour, and the like.

In surimi (water-washed minced fish meat) and minced meat, the decomposition of TMAO (trimethylamine-N-oxide) into DMA (dimethylamine) and formaldehyde during frozen storage is suppressed. Formaldehyde is known to cause freezing denaturation. Decomposition of TMAO in frozen fish meat is thought to be promoted by cryoconcentration due to repeated recrystallization during freezing. As a result, decomposition of TMAO is thought to be suppressed by the recrystallization inhibition activity for ice crystals of the ice nucleation protein of the present invention.

The protein of the present invention has ice nucleation activity and recrystallization inhibition activity for ice crystals. Thus by addition to food, deterioration of the food due to freezing can be suppressed. Although the concentration during addition to food depends on the type of food and the object of use, the concentration added to the total food (at a degree of purity about that of the present protein sample 1 of the working example 1) is about 0.00001% to 10% and preferably is about 0.001% to 0.1%.

Although working examples of the present invention are mentioned below, the present invention is not limited to these working examples.

[Working Example 1]

<Extraction and Purification of Protein Having Ice Nucleation Activity from Antarctic Krill (Whole)>

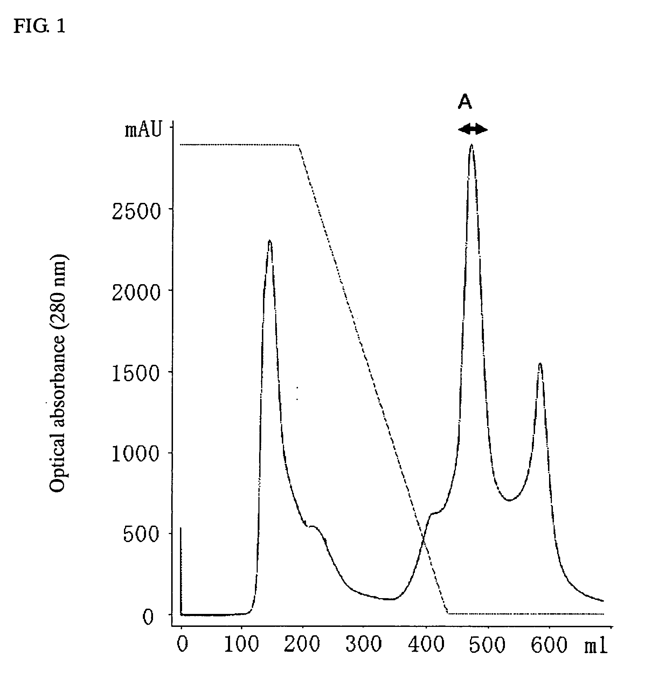

80 g of frozen Antarctic krill was suspended in 420 mL of an extraction liquid containing 50 mM ammonium hydrogen carbonate (pH 7.9) and 1 mM PMSF. Triton-X100 (produced by Sigma Corp.) was added to this suspension liquid to give a concentration of 0.1%, and the mixture was stirred on ice for 1 h. Thereafter, this suspension liquid was subjected to centrifugal separation for 30 minutes at 10,000 G, and the obtained supernatant was subjected to ammonium sulfate fractionation. That is to say, ammonium sulfate at 35 to 65% of saturation was added, and the obtained precipitate was centrifuged for 30 minutes at 10,000 G for separation and recovery. A solution containing 1 M ammonium sulfate and 50 mM ammonium hydrogen carbonate (pH 7.9) (solution A) was added to this precipitate to form a suspension liquid. The supernatant obtained by 20 minutes of centrifuging this suspension liquid at 10,000 G was applied for chromatography using a hydrophobic interaction column (TOYOPEARL Phenyl 650M, Tosoh Bioscience, Inc.; 2.6 cm × 20 cm (106 mL)) equilibrated with the solution A buffer solution, and protein was eluted by a linear concentration gradient using 50 mM ammonium hydrogen carbonate (pH 7.9) (solution B). As a result, the A peak chromatography fraction shown in FIG. 1 was obtained.

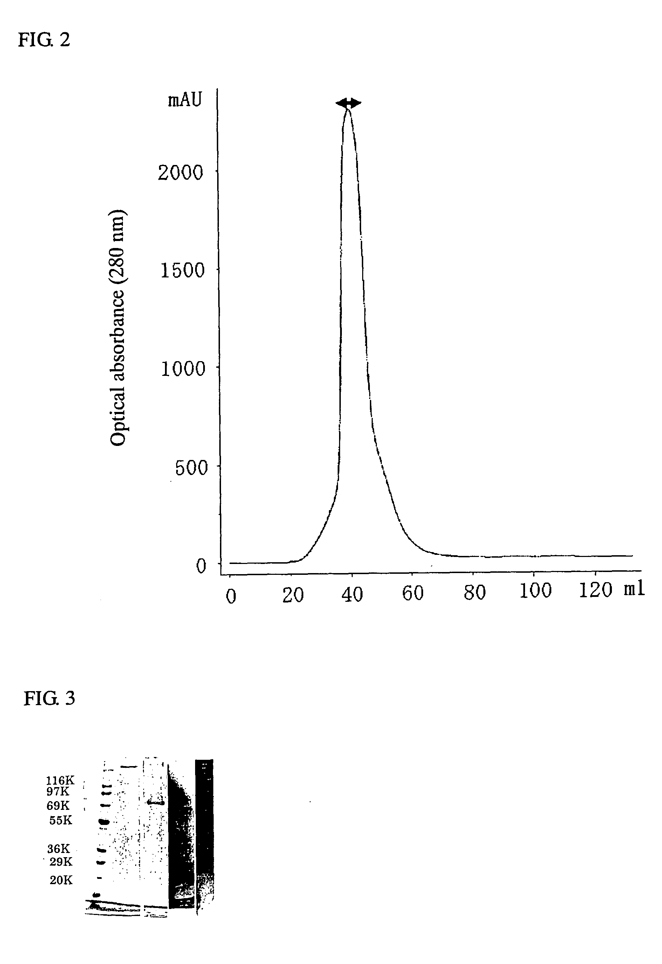

This fraction was placed in a dialysis membrane, the periphery was coated with polyethylene glycol 6000 (produced by Wako Pure Chemical Industries, Ltd.), the fraction was left for about 3 h at 4°C, and the fraction was concentrated roughly 10 fold. Then the resultant fraction was dialyzed against 10 mM ammonium hydrogen carbonate (pH 7.9) overnight at 4°C. After dialysis, the sample was applied for 1.6 cm × 60 cm Sephacryl S-200 HR gel filtration column chromatography (equilibrated beforehand with the same solution). The fraction of the peak indicated by the arrow in the chromatograph shown in FIG. 2 was obtained. This fraction was dialyzed against distilled water. Thereafter, the fraction was subjected to freeze drying. By use of this purification method, 80 mg of protein (present protein sample 1) was obtained from 80 g of frozen Antarctic krill.

[Working Example 2]

<Check of Molecular Weight and the Like Properties of the Present Protein Sample 1>

Molecular weight of the present protein sample 1 was measured by SDS-polyacrylamide gel electrophoresis. A 10% acrylamide gel and a buffer solution (pH 8.6) containing 0.1% SDS, 25 mM tris-hydroxymethyl aminomethane, and 192 mM glycine were used for about 2 h of electrophoresis at 12 mA. Thereafter, proteins were stained using CBB R-250. As a result, this protein was shown to have a subunit structure of about 200 kDa comprising monomers of about 86 kDa or 90 kDa (leftmost column of FIG. 3 shows molecular weight markers; the second column from the left shows the 200 kDa subunit; the third column from the left shows the monomers at 86 kDa or 90 kDa). After the SDS-polyacrylamide gel electrophoresis, the presence of sugar chains was checked by immersion of the gel for 1 h at room temperature in 7.5% acetic acid solution. Thereafter, the gel was transferred to 0.2% periodic acid, and the gel was incubated for 45 minutes at 4°C. Then the gel was transferred to Schiff reagent (Wako Pure Chemical Industries, Ltd.), and the gel was freezed for 45 minutes. Thereafter, the Schiff reagent was removed, and the gel was washed using 10% acetic acid. As a result, the bands at 86 kDa, 90 kDa, and 200 kDa developed coloration showing that sugars were attached to these proteins (second column from the right in FIG. 3). Nile Blue A was used to check for fats. 0.25 g was dissolved in 100 mL of distilled water. Then 1 mL of concentrated sulfuric acid was added, and the mixture was boiled for 2 h. The mixture was filtered through a filter to produce the Nile Blue A solution. The post-electrophoresis gel was transferred to this solution, and the gel was incubated for 30 minutes at 50°C. Thereafter, the gel was transferred to a 5% acetic acid solution, and the gel was incubated for 2 days at 50°C. Then the gel was further incubated for 5 minutes in 0.5% hydrochloric acid solution, and the gel was then washed using distilled water. As a result, bands at 86 kDa, 90 kDa, and 200 kDa developed coloration showing that fatty acids were bonded to these proteins (rightmost column of FIG. 3).

<Amino Acid Sequence Analysis>

The N-terminal amino acid sequence of the present protein sample 1 was analyzed. That is to say, after SDS-polyacrylamide gel electrophoresis was performed in the same manner as that of the method for checking molecular weight, a semi-dry type transfer apparatus was used for transfer to a polyvinylidene fluoride membrane. The bands of the present protein sample 1 were cut out, and the N-terminal amino acid sequences were analyzed using a protein sequencer (model 473A protein sequencer, produced by Applied Biosystems). As a result, the N-terminal amino acid sequences of the present protein sample were found to be those indicated by Protein SEQ ID Nos. 1 and 2 of the Sequence Listing. From this fact, isoforms having similar amino acid sequences are shown to be present in the present protein sample 1.

[Working Example 3]

<Measurement of Ice Nucleation Activity>

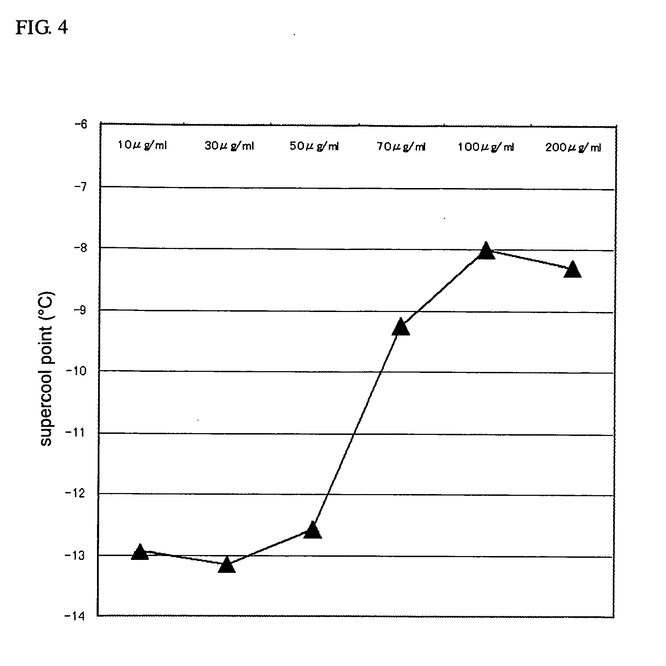

Ice nucleation activity of the above mentioned present protein sample 1 was measured by the below listed method. 100 $\mu$L of protein solution was added to 10 mL of tap water. After 10 minutes of incubation in a constant temperature tank set beforehand to 5°C, temperature was lowered at a rate of 0.3°C/minute, and the freezing temperature (supercooling temperature) was measured.
As a result, as shown in FIG. 4, supercooling temperature increased in a concentration-dependent manner at 0 to 200 $\mu$g/mL protein concentrations, and supercooling temperature rose to -8°C at 100 $\mu$g/mL. The supercooling temperature of tap water (as a control) was -13°C. Thus the present protein sample 1 was shown to be a protein that had ice nucleation activity that elevated the supercooling temperature.

<Temperature Stability of Ice Nucleation Activity>

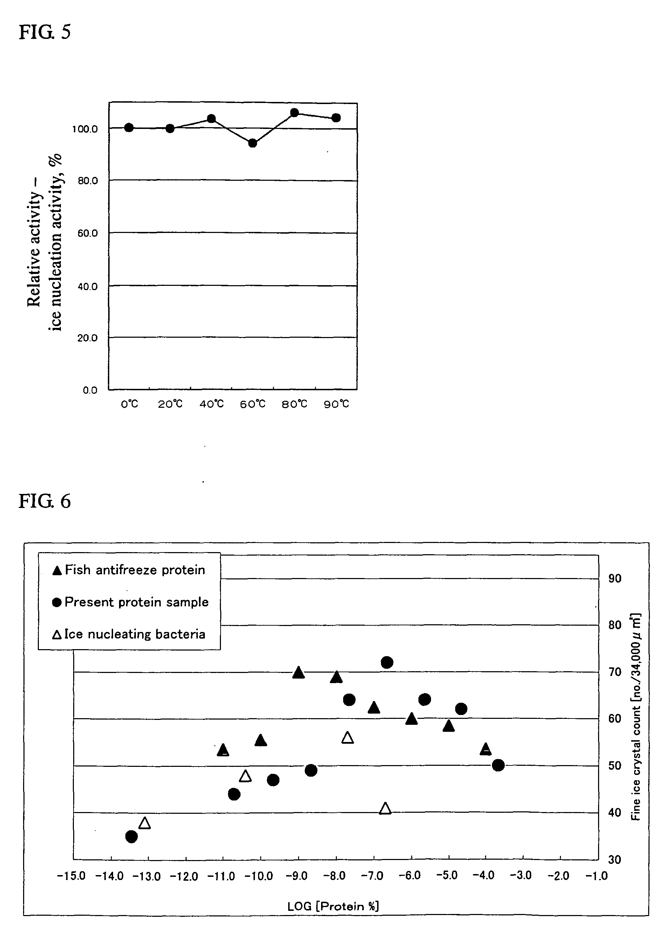

Temperature stability of the present protein sample 1 was tested. Samples were dissolved in 50 mM ammonium hydrogen carbonate aqueous solutions (pH 7.9, 7.5 mg/mL protein concentration), and these solutions were held for 1 h at respective temperatures (0°C to 90°C). Thereafter, ice nucleation activity was measured. As a result, 90 to 106% of ice nucleation activity was found be to retained relative to activity of the sample held at 0°C, and stability with respect to heat was shown (FIG. 5).

[Working Example 4]

<Measurement of Recrystallization Inhibition Activity for Ice Crystals>

Ice crystals were observed using a microscope, and the count of fine ice crystals was used as the recrystallization inhibition activity for ice crystals of the present protein sample 1. That is to say, 2 $\mu$l of the present protein sample dissolved in 30% sucrose solution was placed drop-wise on a cover glass, and another cover glass was placed thereon to sandwich the solution therebetween. The cover glass assembly was placed on the stage of an optical microscope (model BH2 microscope, produced by Olympus Corp.; and LK600 temperature controller, produced by Linkam Scientific Instruments Ltd.) that was capable of temperature control, and heating and cooling were performed repeatedly at a rate of 0.1°C/second, in order, to temperatures of 20°C, -30°C, 20°C, -30°C, 20°C, -30°C, and -10°C. Then temperature was held at -10°C for 30 minutes, and the count was determined of fine ice crystals having a surface area of 10.01 to 35 $\mu m^2$. As a result, as shown in FIG. 6, the present protein sample 1 was confirmed to have nearly the same recrystallization inhibition activity for ice crystals as fish antifreeze protein (A/F Protein Inc., AFP type III) at concentrations of $2.2 \times 10^{-10}$ to $2.2 \times 10^{-2}$ mg/mL despite not having antifreeze activity.
Testing was performed to find whether or not other proteins having ice nucleation activity also had this type of recrystallization inhibition activity for ice crystals. Similar testing was performed using "crushed cells, derived from the genus Xanthomonas (Wako Pure Chemical Industries, Ltd.)," which are ice nucleating bacteria, as the sample. As shown in FIG. 6, recrystallization inhibition activity for ice crystals was found also for the bacteria-derived ice nucleation protein. Although no example has been reported heretofore of a protein having ice nucleation activity that had recrystallization inhibition activity, the ice nucleation protein is hypothesized to limit the size of individual ice crystals and to affect size of the final ice crystals by producing a large number of ice nuclei at the time of ice nuclei formation. The possession of recrystallization inhibition activity for ice crystals by this ice nucleation protein is a new finding.

[Working Example 5]

<Purification of Proteins Having Ice Nucleation Activity from Various Parts of Antarctic Krill>

In order to check what part of the krill contains a large amount of the above mentioned protein having ice nucleation activity, eyeballs, meat, liver/pancreas, and shell were sorted, and the presence of protein was checked for each of the parts using SDS-polyacrylamide gel electrophoresis. Since a particularly large amount was found to be contained in the shell, 20 mg of protein was obtained from 10 g of shell of Antarctic krill using the same purification method as that of working example 1.

[Working Example 6]

<Measurement of Ice Nucleation Activity of Crustacean-Derived Protein>

Ice nucleation activity of crustacean-derived protein was observed for non-krill crustaceans. Using the same method as that of working example 1, protein was purified from the shells of king crab, snow crab, and American crayfish. As a result of performance of observations, ice nucleation activity was confirmed (Table 1).
[Table 1]

|  | Raw material weight (g) | Extraction amount (mg) | Yield (%) | Antifreeze activity (°C) *1 | Ice nucleation activity (°C) *2 |
|---|---|---|---|---|---|
| Antarctic krill (shell) | 10 | 20 | 0.2 | 0.4 | -6.83 |
| snow crab (shell) | 20 | 2 | 0.01 | 0.48 | -9.2 |
| king crab (shell) | 30 | 3 | 0.01 | 0.24 | -6.83 |
| American crayfish (shell) | 12 | 6 | 0.05 | 0.3 | -5.6 |
| *1: Protein concentration = 1 mg/mL. *2: Protein concentration = 0.1 mg/mL. | | | | | |

[Working Example 7]

<Hot Water Extraction of Protein Having Ice Nucleation Activity from Antarctic Krill>

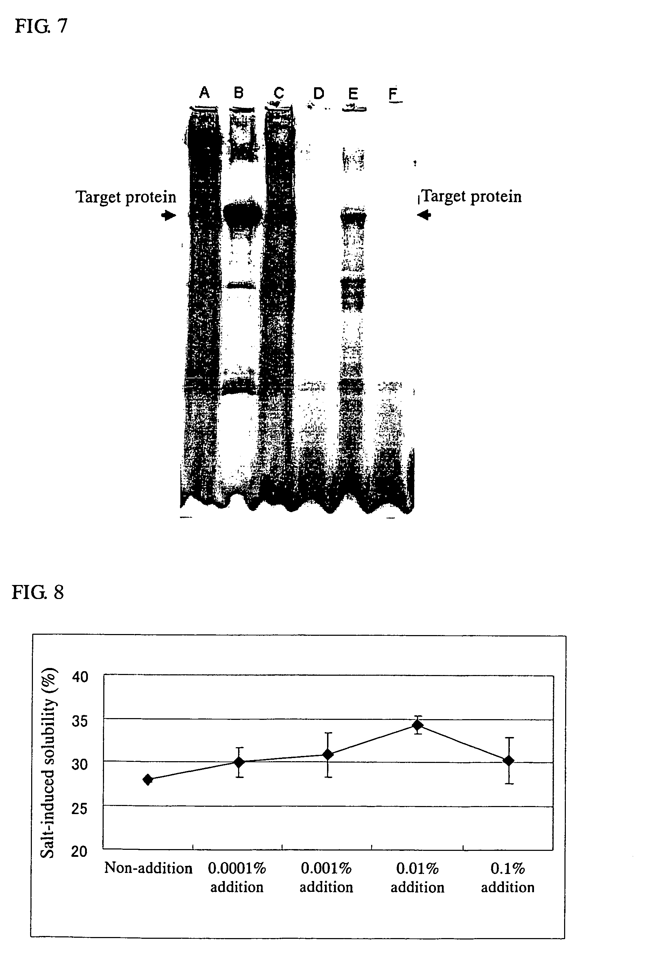

15 g of frozen Antarctic krill was pulverized using a homogenizer, and 15 g of water was added to make a suspension. After incubation of such mixtures for 30 minutes, each under different temperature conditions (50°C, 80°C, 100°C), extracts were produced as the incubates. Then, the obtained extracts were subjected to centrifugal separation (3 minutes at 15,000 G), and the obtained extract liquids were analyzed by SDS-polyacrylamide gel electrophoresis. Electrophoresis results as shown in FIG. 7. The 50°C extract was applied to lane A. The centrifugal supernatant of the 50°C extract was applied to lane B. The 80°C extract was applied to lane C. The centrifugal supernatant of the 80°C extract was applied to lane D. The 100°C extract was applied to lane E. The centrifugal supernatant of the 100°C extract was applied to lane F. As shown in FIG. 7, the target protein was almost not found in the extracts at temperatures greater than or equal to 80°C. However, the target was contained at a large concentration in the centrifugal supernatant of the 50°C extract, and the possibility of efficient extraction was confirmed.

[Working Example 8]

<Application 1 to Food: Addition to Seafood - Minced meat of Alaska Pollock>

Meat was collected from Alaska pollock, which is a fresh fish caught off of the Sanriku Coast, and a 3 mm mesh mincer was used to form ground meat. Then 500 g of this ground meat was sampled, and 50 g of water and 50 g of sugar were added. The present protein sample 1 was further added to give concentrations ranging from 0 to 0.1%. The mixture was stirred for 25 seconds using a tabletop mixer. This mixture was then used as the minced meat sample.
Then the respective samples were frozen overnight at -25°C and were stored thereafter for 2 weeks at -10°C. These samples were used for measurement of salt-induced solubility and degree of TMAO decomposition as indicators for evaluation of preservation ability.

Measurement of Salt-Induced Solubility

After addition of 27 mL of 0.1 M KCl 20 mM Tris-HCl (Ph 7.5) solution to 3 g of minced meat, the mixture was homogenized using a homogenizer (10,000 rpm × 30 seconds, 3 times). Thereafter, the mixture was subjected to centrifugal separation (5,000 rpm × 10 minutes) to obtain a precipitate. Then an additional 27 mL of the same solution was added to this precipitate, and the mixture was stirred. Centrifugal separation was performed again (5,000 rpm × 10 minutes), and 27 mL of 0.5 M KCl 20 mM Tris-HCl (pH 7.5) solution was added to the obtained precipitate. The mixture was stirred, 30 μL of 1 M ATP was added, and the mixture was stored overnight on ice. The mixture thereafter was stirred, volume of the suspension solution was measured, and protein concentration of the suspension solution was measured by the biuret method. The suspension solution was subjected to further centrifugal separation (7,000 rpm × 15 minutes), and protein concentration of the obtained supernatant was measured by the biuret method. Volume was also measured. Based on the above measurements, salt-induced solubility was calculated from the ratio of the protein concentration in the previous supernatant over the protein concentration in the suspension liquid. As a result, as shown in FIG. 8, salt-induced solubility increased with increased added amount of the present protein sample 1, and due to addition of the present protein sample 1, a trend was shown for suppression of deterioration of minced meat of Alaska pollock during frozen storage.

Measurement of Decomposition Reaction of TMAO

After 10 mL of distilled water and 10 mL of 10% trichloroacetic acid (TCA) were added to 10 g of the minced meat sample, the mixture was homogenized (12,000 rpm × 2 minutes). Thereafter, centrifugal separation (7,500 rpm × 20 minutes) was used to obtain a supernatant. Then 5 mL of 5% TCA solution was added to the precipitate, the mixture was well stirred, and centrifugal separation was used to obtain a supernatant. This operation was carried out 2 times. The heretofore obtained supernatants were placed in a 50 mL measuring flask, and 5% TCA solution was used to increase volume to the meniscus mark. This solution was used as the TCA extract liquid.
Then 1 mL of the TCA extract liquid and 3 mL of distilled water were mixed, 0.4 mL of copper-ammonia reagent and 5% carbon disulfide - benzene solution were added, and the mixture was incubated for 5 minutes at 40°C. Thereafter, the mixture was repeatedly shaken, and centrifugal separation (3,500 rpm × 5 minutes) was performed. Then the obtained supernatant was transferred to a test tube containing about 0.2 g of anhydrous sodium sulfate, and optical absorbance of this solution was measured at 440 nm. The generated concentration of DMA is indicated by the following formula.

```
DMA amount (mM) = (value measured at 440 nm) ÷ 1.3 × 2 × 50 ÷

1000 ÷ (sample weight) × 1000
```

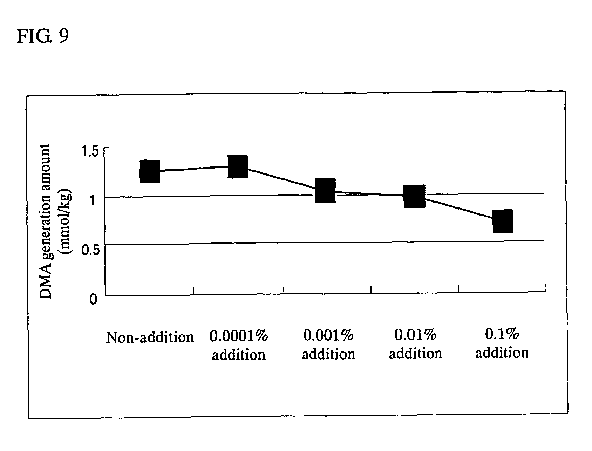

As shown in FIG. 9, the generated concentration of DMA due to decomposition of TMAO was shown to decline according to the added concentration of the present protein sample 1. Due to addition of the present protein sample 1, formaldehyde is generated at the same amount as DMA due to decomposition of TMAO. Suppression of the generated amount of DMA means suppression of generation of formaldehyde, and this indicates that freezing denaturation of Alaska pollock minced meat by formaldehyde was suppressed during frozen storage.

[Working Example 9]

<Application 2 to Food: Raw Whole Chicken Egg>

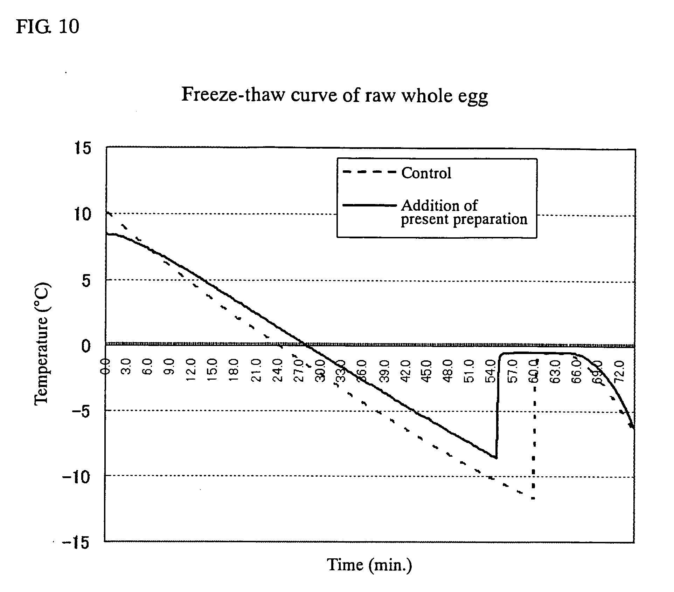

0.56 mg (protein concentration) of the present protein sample 1 was added to 30 mL of raw chicken egg. Thereafter, this whole egg was placed in a polyvinylidene chloride type casing (Krehalon DB56R) and was stored for 1 day at -20°C. Thermal history was measured during freezing (FIG. 10). After freezing, the sample was thawed at room temperature and heated in boiling water to produce a gel. As a result, addition of the present preparation was found to raise the supercooling temperature to -8.56°C, in contrast to -11°C of the control. Although the whole egg of the control was immediately fluidized after thawing, the whole egg to which the present preparation was added had viscosity nearly the same as that of the non-frozen egg. When a cross section of the heated gel was observed by scanning electron microscope, the network of the gel was found to be dense for the egg to which the present preparation had been added. Quality of frozen whole egg could be increased by addition of the present preparation.

[Working Example 10]

<Application 3 to Food: Surimi (water-washed minced fish meat)>

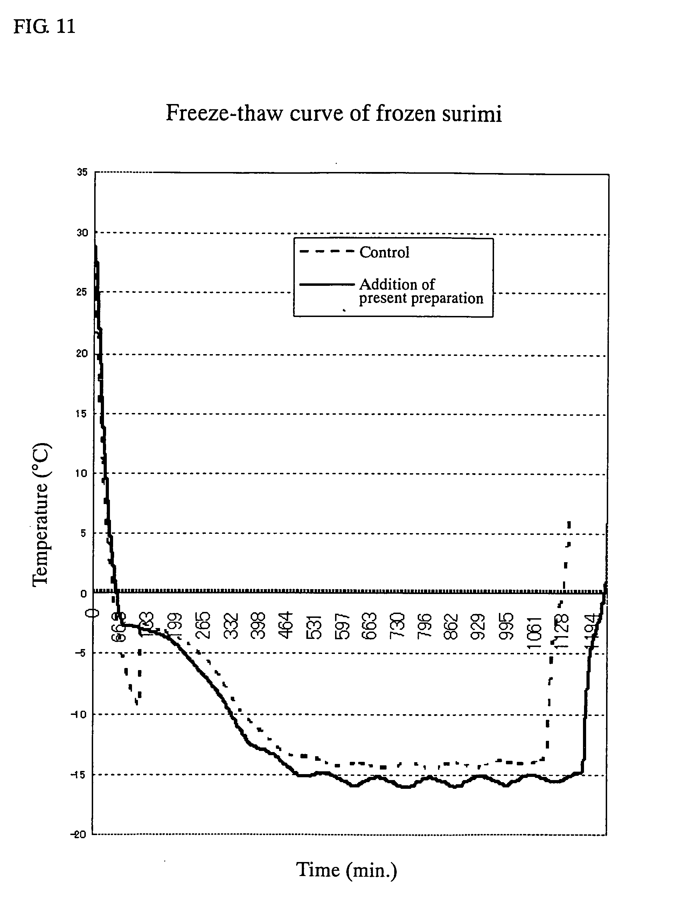

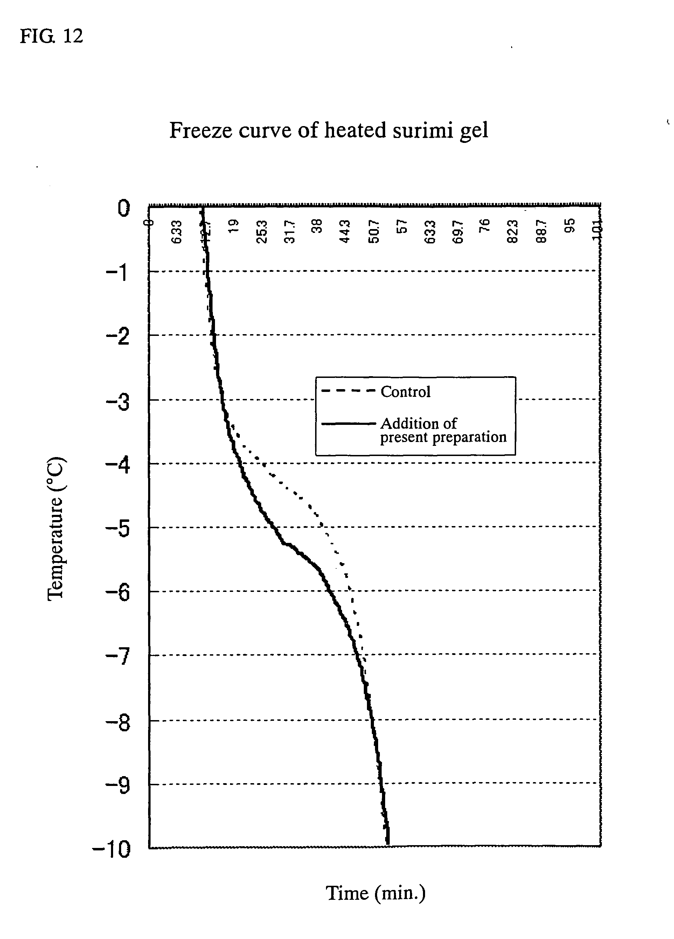

0.56 mg (protein) of the present protein sample 1 was added to 30 g of frozen surimi of Alaska pollock, and the mixture was mixed using a food cutter. Thereafter, this surimi was placed in a polyvinylidene chloride type casing (Krehalon DB56R) and was stored overnight at -20°C. Then the surimi was thawed. Temperature history was measured during the freezing and thawing (FIG. 11). After thawing, the frozen fish meat in the casing was heated for 30 minutes in boiling water. As a result, although supercooling in the vicinity of -9°C (freezing temperature) occurred for the control, freezing started in the vicinity of -3°C when the present preparation was added, and further supercooling did not occur. The surimi containing the present preparation began to thaw at room temperature (25°C) with a delay of about 1 h and 30 minutes. Also, looking at the freezing curve (FIG. 12) of the heated gels of these surimi, the time of passage through the maximum ice crystal growth region (-1°C to -5°C) was found to be about 10 minutes faster when the present preparation was added.

[INDUSTRIAL APPLICABILITY]

The protein of the present invention is contained in large amounts in shells of crustaceans which are mostly discarded except for use as meal, chitin, and chitosan raw material. Thus large volume preparation, as well as inexpensive production, is possible. Moreover, application by addition to foods is easy since this is a food-derived protein. Since the protein of the present invention has ice nucleation activity and recrystallization inhibition activity for ice crystals, wide use is possible with the object of quality maintenance and the like of frozen food and the like.

EP 2 006 295 A2

[Sequence Listing Free Text]

SEQ ID No.: 1

N-terminal amino acid sequence

SEQ ID No.: 2

N-terminal amino acid sequence

[Sequence Listing]

<110> Nippon Suisan Kaisha, Ltd.

<120> Protein having ice nucleation activity

<130> YCT-1257

<141> 2007-03-13

<150> JP 2006-67758

<151> 2006-03-13

<160> 2

<210> 1

<211> 20

<212> PRT

<213> Antarctic krill (Euphausiacea sp.)

<400> DYDVAHEQQDVNAFLTKITG (one letter code)

　　　　Asp Tyr Asp Val Ala His Glu Gln Gln Asp Val Asn Ala Phe

Leu Thr Lys Ile Thr Gly (three letter code)

<210> 2

<211> 18

<212> PRT

<213> Antarctic krill (Euphausiacea sp.)

<400> SDPKFQQDINTRLXNVYE (one letter code)

　　　　　Ser Asp Pro Lys Phe Gln Gln Asp Ile Asn Thr Arg Leu

Xaa Asn Val Tyr Glu (three letter code)

11

**Claims**

1. A method for inhibition of recrystallization of ice crystals using a protein having ice nucleation activity.

2. The method for inhibition of recrystallization of ice crystals according to claim 1, wherein the protein having ice nucleation activity is a protein derived from a crustacean.

3. A crustacean-derived protein having ice nucleation activity.

4. The protein having ice nucleation activity according to claim 3; wherein
   the crustacean-derived protein, as measured by molecular weight measurement using sodium dodecyl sulfate (SDS) - polyacrylamide gel electrophoresis, has a non-reduced-form molecular weight of about 200,000 and displays reduced-form bands at about 86,000 and 90,000; and
   the N-terminal amino acids are indicated by SEQ ID No. 1 or SEQ ID No. 2.

5. The protein having ice nucleation activity according to claim 3 or claim 4; wherein the crustacean is a shrimp, crayfish, crab, krill, or mantis shrimp.

6. A crustacean extract having ice nucleation activity and including a crustacean-derived protein having ice nucleation activity.

7. The crustacean extract having ice nucleation activity according to claim 6; wherein
   the crustacean-derived protein having ice nucleation activity, as measured by molecular weight measurement using sodium dodecyl sulfate (SDS) - polyacrylamide gel electrophoresis, has a non-reduced-form molecular weight of about 200,000 and displays reduced-form bands at about 86,000 and 90,000; and
   the N-terminal amino acids are indicated by SEQ ID No. 1 or SEQ ID No. 2.

8. The crustacean extract having ice nucleation activity according to claim 6 or claim 7; wherein the crustacean is a shrimp, crayfish, crab, krill, or mantis shrimp.

9. A production method for a crustacean extract having crustacean-derived ice nucleation activity; wherein the following is utilized with or without further purification:

   a liquid obtained by crushing meat and/or internal organs of the crustacean; or
   an extract liquid of the shell, meat, and/or internal organs.

10. The production method of a crustacean extract having crustacean-derived ice nucleation activity according to claim 9; wherein
    purification is used to concentrate a protein, as measured by molecular weight measurement using sodium dodecyl sulfate (SDS) - polyacrylamide gel electrophoresis, having a non-reduced-form molecular weight of about 200,000, and displaying reduced-form bands at about 86,000 and 90,000; and
    having the N-terminal amino acids as indicated by SEQ ID No. 1 or SEQ ID No. 2.

11. The production method of a crustacean extract having crustacean-derived ice nucleation activity according to claim 9 or claim 10; wherein an extraction liquid containing added surfactant is used for extraction from the shell, meat, and/or internal organs of the crustacean.

12. The production method of a crustacean extract having crustacean-derived ice nucleation activity according to claim 9, 10, or 11; wherein the crustacean is a shrimp, crayfish, crab, krill, or mantis shrimp.

13. A method for suppression of quality deterioration due to freezing of food by suppressing recrystallization of ice crystals; wherein a protein is added that has ice nucleation activity.

14. The method of claim 13; wherein
    the protein of any one of claims 3 to 5 is added as the protein having ice nucleation activity; or
    the crustacean extract of any one of claims 6 to 8 is added.

15. The method of claim 13 or claim 14; wherein the food is a frozen food.

16. The method according to claim 13, 14, or 15; wherein the frozen food is a food utilizing egg, wheat flour, or fish meat as a raw material.

17. The method according to claim 16; wherein the frozen food is surimi or minced fish meat.

18. A food quality improvement agent including:

   the protein having ice nucleation activity according to any one of claims 1 to 5;
   the crustacean extract having ice nucleation activity according to any one of claims 6 to 8; or
   the protein having ice nucleation activity produced by the method of any one of claims 9 to 12.

19. A food having improved quality and to which has been added the quality improvement agent of claim 18.

20. The food according to claim 19; wherein the food is a frozen food.

21. The food according to claim 19 or claim 20; wherein the food is a food utilizing egg, wheat flour, or fish meat as a raw material.

FIG. 1

FIG. 2

FIG. 3

FIG. 4

FIG. 5

FIG. 6

## FIG. 7

Target protein →

← ₁Target protein

## FIG. 8

FIG. 9

FIG. 10

Freeze-thaw curve of raw whole egg

FIG. 11

Freeze-thaw curve of frozen surimi

Time (min.)

FIG. 12

Freeze curve of heated surimi gel

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO H6181729 A
- WO 3028246 A
- WO H6113712 A
- WO 3090932 A

**Non-patent literature cited in the description**

- *Appl. Microbiol.,* 1974, vol. 28, 456
- *Proc. 4th Int. Cont. Plant. Path. Bact.,* 1978, 725